# EUROPÄISCHE PATENTSCHRIFT

(11) **EP 0 989 155 B1**
(45) Veröffentlichungstag und Bekanntmachung des Hinweises auf die Patenterteilung: **30.03.2005**
(21) Anmeldenummer: 99117178.6
(22) Anmeldetag: 01.09.1999
(51) Int. Cl.: C08J 3/215, C08L 21/00

(54) **Pulverförmige, modifizierte Füllstoffe enthaltende Kautschukpulver, Verfahren zu ihrer Herstellung und Verwendung**
Powdery modified fillers containing rubber powder, method for the production and use thereof
Matières de charge modifiées sous forme de poudre, contenant de la poudre de caoutchouc, leur procédé de production et leur utilisation

(30) Priorität: 22.09.1998 DE 19843301
(43) Veröffentlichungstag der Anmeldung: 29.03.2000
(73) Patentinhaber: PKU Pulverkautschuk Union GmbH, 45764 Marl (DE)
(72) Erfinder: Görl, Udo, Dr., 53332 Bornheim-Roisdorf (DE); Stober, Reinhard, Dr., 63594 Hasselroth (DE); Lauer, Hartmut, 63628 Bad Soden-Salmünster (DE); Ernst, Uwe, 45764 Marl (DE)

(56) Entgegenhaltungen:
- EP-A- 0 753 549
- DE-A- 3 723 213
- DE-A- 4 013 005

## Beschreibung

Die Erfindung betrifft ein Verfahren zur Herstellung von mit Organosiliciumverbindungen modifizierte Füllstoffe enthaltenden Kautschukpulvern und die so hergestellten Pulver.

Über Ziel und Zweck des Einsatzes von Pulverkautschuken, sowie über mögliche Verfahren zu ihrer Herstellung ist eine Vielzahl von Publikationen erschienen.

Die Erklärung für das Interesse an pulverförmigen Kautschuken ergibt sich zwanglos aus der Verarbeitungstechnik der Gummiindustrie. Dort werden die Kautschukmischungen mit einem hohen Aufwand an Zeit, Energie und Personal hergestellt. Hauptgrund dafür ist, daß der Rohstoff Kautschuk ballenförmig vorliegt und die weiteren Bestandteile der vulkanisierbaren Mischung eingearbeitet werden müssen.

Die Zerkleinerung des Ballens, die innige Vermischung mit Füllstoffen, Mineralölweichmachern und Vulkanisationshilfsmitteln erfolgt auf Walzen oder in Innenmischern in mehreren Verfahrensstufen. Zwischen den Stufen wird die Mischung im allgemeinen auf einer Batch-off-Anlage abgekühlt, als Fell auf Paletten abgelegt und zwischengelagert. Den Innenmischern bzw. Walzen werden entsprechende Extruder oder Kalendrierprozesse nachgeschaltet.
Aus dieser sehr aufwendigen Technik der Kautschuk-Verarbeitung kann nur eine völlig neue Verarbeitungstechnologie herausführen.
Es wird daher seit längerem der Einsatz rieselfähiger Kautschukpulver diskutiert, weil sich damit die Möglichkeit ergibt, Kautschukmischungen wie thermoplastische Kunststoffpulver einfach und schnell verarbeiten zu können.

Aus der DE-PS 2822 148 ist ein Verfahren zur Herstellung eines pulverförmigen, füllstoffhaltigen Kautschuks bekannt.

Man setzt gemäß dieser Patentschrift einem Kautschuk-Latex (z. B. Naturkautschuk), einer Kautschuk-Lösung (z. B. BR) oder einer wäßrigen Emulsion eines synthetischen Kautschuks (z. B. SBR) eine wäßrige Füllstoffemulsion zu und fällt das gewünschte Kautschukpulver aus.
Um die nach diesem Verfahren erhaltenen, korngrößenabhängigen Füllstoffgehalte zu vermeiden, wurden Varianten angemeldet, die als DE-PS 3723 213 und DE-PS 3723 214 zum Stand der Technik gehören.
Gemäß DE-PS 3723213 wird in einem zweistufig ablaufenden Verfahren zuerst eine Menge ≥50 % des Füllstoffs in das Kautschukpulverpartikel integriert. Im zweiten Schritt wird oder Rest des Füllstoffs auf das sogenannte Kautschukgrundkorn aufgezogen.
Dies kann als eine Variante des Puderns angesehen werden, da keine Bindung zwischen Füllstoff und Kautschuk entsteht.

Wie *E.T. Italiaander* (Vortrag 151. Technische Tagung der Rubber Div. der ACS, Anaheim, Kalifornien, 6. - 9. Mai 1997(GAK 6/1997 (50) 456-464)aber feststellt, ist ungeachtet der großen Zukunft, die im Delphi-Report (Delphi Report Künftige Herstellverfahren in der Gummiindustrie" Rubber Journal, Vol. 154, Nr. 11, 20-34 (1972)) für pulverförmigen und granulierten Kautschuk vorausgesagt wurde, und zahlreicher Versuche, die von namhaften Polymerherstellern ab Mitte der siebziger Jahre bis in die frühen achtziger Jahre zur Herstellung von pulverförmigen NBR, SBR-Ruß-Masterbatches und granuliertem NR unternommen wurden, die Standard-Lieferform von Polymeren der Kautschukballen geblieben.

Ein Nachteil der bekannten Verfahren liegt zum einen darin, daß für die Einstellung des für die Qualität des Endprodukts als notwendig erachteten Korngrößendurchmessers der Füllstoffteilchen 10 µm ein Mahlvorgang notwendig ist.

Dieser bedingt aber nicht nur einen hohen Energieaufwand, sondern verursacht auch eine Schädigung der Füllstoffstruktur, die neben der aktiven Oberfläche eine wichtige Kenngröße für die Wirksamkeit in der Gummianwendung darstellt.

Zum anderen leidet die Handhabbarkeit der Produkte nach dem Stand der Technik darunter, daß die Partikel bei der Lagerung miteinander verkleben.

Unter der Nummer P 198 16 972.8 wurde jetzt ein Fällverfahren angemeldet, bei dem Suspensionen von mit Organosiliciumverbindungen modifizierten Füllstoffen hergestellt und diese in die Kautschukemulsion eingerührt werden.
Aus diesem Gemisch wird anschließend das Kautschukpulver ausgefällt.

Aufgabe der Erfindung ist es, ein Verfahren bereitzustellen, das mit weniger Verfahrensstufen zu einem vorteilhaft einzusetzenden Kautschukpulver führt, das modifizierte Füllstoffe enthält.

Gegenstand der Erfindung ist ein Verfahren zur Herstellung feinteiliger Kautschuke (Kautschukpulver) durch Ausfällen aus wasserhaltigen Mischungen, die Füllstoff in Form von Suspensionen, wasserlösliche Salze eines Metalls der Gruppen IIa, IIb, IIIa und VIII des periodischen Systems der Elemente und einen Kautschuklatex, eine wässrige Emulsion eines Kautschuks oder eine Kautschuklösung enthalten, das dadurch gekennzeichnet ist, daß man
a) eine oder mehrere Organosiliciumverbindung(en), die mindestens eine Alkoxygruppe enthalten, in Wasser gelöst oder gegebenenfalls in Anwesenheit einer oberflächenaktiven Substanz emulgiert, oder die genannten Verbindungen direkt, gegebenenfalls mit einer oberflächenaktiven Substanz mit der wässrigen Suspension eines oxidischen oder silikatischen feinteiligen Füllstoffs oder einer Mischung dieser Füllstoffe mit einem mittleren Teilchendurchmesser < 10 µm bei einer Temperatur von 10 bis 60 ° C, bevorzugt bei Raumtemperatur, unter Rühren vermischt, wobei man die für die Einarbeitung in den Kautschuk vorgesehene Menge dieses Gemisches, bezogen auf den Füllstoffanteil in zwei Partien aufteilt, und
b) eine erste Partie mit dem Polymerlatex, der Polymeremulsion bzw. der Polymerlösung vermischt, den pH-Wert dieser Mischung mit einer Säure, insbesondere einer Lewissäure, auf einen pH-Wert von 6,0 bis 4,5 absenkt (erste Partie, erste Stufe),
c) den restlichen Anteil (zweite Partie, Splittinganteil) unter weiterer Absenkung des pH-Wertes auf 4,5 bis 2,6 insbesondere ca. 3,2 zusetzt (zweite Stufe), sodaß der in der Mischung befindliche Kautschuk zusammen mit dem durch Organosiliciumverbindung(en) modifizierten Füllstoff ausfällt
d) den ausgefallenen Feststoff mit an sich bekannten Maßnahmen abtrennt,
e) ihn bevorzugt wäscht, um den pH-Wert auf einen für die weitere Verarbeitung verträglicheren Wert von ca. 6 bis 7 einzustellen, und
f) den füllstoffhaltigen Kautschuk trocknet.

Die Trocknung erfolgt vorteilhaft in einem Trockner bei einer Gaseintrittstemperatur von 140 bis 160°C und einer Gasaustrittstemperatur von 50 bis 70°C. Die Temperatur des Produkts sollte 40 bis 50 °C nicht überschreiten.

Die von dem pH-Wert und dem Füllstoffgehalt abhängige Dauer und der Umfang des Ausfällvorgangs kann innnerhalb einer Meßreihe einfach festgestellt werden.

Bei einem Pulverkautschuk mit hohem Füllungsgrad (≥80 Teile Füllstoff phr) wird man im allgemeinen 1 bis 10 Teile dieser Menge als restlichen Anteil in der zweiten Stufe bei der Ausfällung des Pulverkautschuks einsetzen.

Enthält der Pulverkautschuk weniger als 80 Teile Füllstoff phr, z. B. nur insgesamt 50 Teile phr, führt man vor dem Abschluß des Fällvorgangs noch >10 bis 20 Teile dieser Menge in Form einer Suspension in die Mischung ein.

Auf diese Weise werden die Füllstoffe in den äußeren Kornbereich (Randbereich) der Kautschukpulver eingebunden.

Diese Anteile des Füllstoffs sind damit nicht äußerlich auf die einzelnen Kautschukpartikel aufgezogen (s. DE-PS 37 23213), sondern in die Kautschukoberfläche integriert.

Diese Füllstoffverteilung und die Art der Bindung der Füllstoffe in der Kautschukmasse bewirken die hohe Fließfähigkeit der erfindungsgemäßen Pulver und verhindern das Verkleben während der Lagerung der Pulver, ohne daß diese Eigenschaften durch mechanische Belastungen beim Fördern, Silieren etc. verlorengehen.

Als weitere Füllstoffe setzt man gegebenenfalls die aus der Gummiindustrie bekannten Ruße, bevorzugt in feinteiliger Form (fluffy) ein, die im allgemeinen ohne mechanische Behandlung einen mittleren Korngrößendurchmesser von 1 bis 9 µm, vorzugsweise 1 bis 8 µm aufweisen, bevor sie suspendiert sind.

Dies erleichtert die Dispersion, so daß man ohne hohen Energieaufwand zu wäßrigen Suspensionen mit Füllstoffpartikeln eines mittleren Teilchendurchmessers deutlich kleiner als 10 µm gelangt.
Gefällte Kieselsäure kann vorteilhaft in Form eines salzfrei gewaschenen Filterkuchens eingesetzt werden.

Als Metallsalze kommen solche in Frage, die von Elementen der Gruppen IIa, IIb, IIIa und VIII des periodischen Systems der Elemente stammen. Diese Gruppeneinteilung entspricht der alten IUPAC-Empfehlung (siehe Periodisches System der Elemente, Verlag Chemie, Weinheim, 1985) Typische Vertreter sind Magnesiumchlorid, Zinksulfat, Aluminiumchlorid, Aluminiumsulfat, Eisenchlorid, Eisensulfat, Kobaltnitrat und Nickelsulfat, wobei die Salze des Aluminiums bevorzugt sind. Besonders bevorzugt sind Aluminiumsulfat und weitere Lewissäuren.
Die Salze werden in einer Menge von 0,1 bis 6,5 Gewichtsteilen pro 100 Gewichtsteile Kautschuk eingesetzt. Die Metallsalze erweisen sich als besonders geeignet, die Korngröße der ausgefällten Produkte in gewünschter Weise zu beeinflussen. Zur Einstellung des gewünschten pH-Wertes verwendet man gegebenenfalls zusätzlich Mineralsäuren, wie z. B. Schwefelsäure, Phosphorsäure und Salzsäure, wobei die Schwefelsäure besonders bevorzugt ist. Eingesetzt werden können aber auch Carbonsäuren, wie z. B. Ameisen- und Essigsäure.

Die Menge an Säure richtet sich nach der Art und Menge des wasserlöslichen Metallsalzes, des Füllstoffs, des eingesetzten Organosilans, des Kautschuks und des gegebenfalls vorhandenen Alkalisilikats. Sie läßt sich durch einige orientierende Versuche leicht ermitteln.

Nach einer bevorzugten Ausführungsform des erfindungsgemäßen Verfahrens werden zusätzlich noch bis zu 5 Gewichtsteile pro 100 Gewichtsteile Kautschuk Kieselsäure (SiO₂) in Form einer Alkalisilikatlösung, vorzugsweise als Wasserglas mit einem Na₂O: SiO₂-Molverhältnis von 2 : 1 bis 1 : 4, eingesetzt. Die Alkalisilikatlösung kann dabei sowohl der Kautschukkomponente als auch der Füllstoff-Suspension zugesetzt werden. Bevorzugt ist die Zugabe zur Kautschukkomponente, besonders bei der kontinuierlichen Fahrweise.

Im allgemeinen wir das erfindungsgernäße Verfahren wie folgt durchgeführt:
Zunächst wird eine Füllstoff-Suspension in der Weise hergestellt, daß man einen Teil, vorzugsweise ≥50 %, des im Endprodukt enthaltenen, Füllstoffs zusammen mit dem Metallsalz, der Organosilanverbindung und gegebenenfalls der Alkalisilikatlösung in Wasser, gegebenenfalls in Gegenwart eines Emulgators, dispergiert. Die Menge des insgesamt eingesetzten Wassers richtet sich nach der Art des Füllstoffs und dem Aufschlußgrad. Im allgemeinen liegen die nicht wasserlöslichen Bestandteile der Suspension bei etwa 4 bis 15 Gewichtsprozent. Dieser Wert stellt keine bindende Beschränkung dar und kann sowohl unter- als auch überschritten werden. Der maximale Gehalt wird durch die Pumpbarkeit der Suspension beschränkt.

Die so hergestellte Füllstoffsuspension wird anschließend mit dem gegebenfalls Alkalisilikatlösung enthaltenden

Kautschuk-Latex oder der gegebenfalls Alkalisilikatlösung enthaltenden wäßrigen Emulsion einer Kautschuk-Lösung innig vermischt (erste Partie, erste Stufe). Dazu eignen sich bekannte Rühraggregate, wie z. B. Propeller-Rührer.

Nach dem Vermischen wird unter weiterem Rühren, bevorzugt einer Lewissäure, insbesondere Al₂(SO₄)₃ mit Hilfe einer Säure zunächst ein pH-Wert im Bereich von 6,0 bis 4,5 eingestellt. Dabei fällt ein Kautschukgrundkorn mit einem konstanten Füllstoff- und Organosilangehalt an. Die Größe dieses Grundkorns wird durch die gewählte Metallsalzmenge im Bereich von 0,1 bis 6,5 phr gesteuert. Die Steuerung vollzieht sich so, daß mit der niedrigsten Menge an Metallsalz die größte Körnung erhalten wird.

Den restlichen Anteil der Füllstoff-Suspension (zweite Partie, Splittinganteil) setzt man unter weiterer Absenkung des pH-Wertes auf 4,5 bis 2,6, insbesondere ca. 3,2 zu (zweite Stufe), sodaß der in der Mischung befindliche Kautschuk zusammen mit dem durch Organosiliciumverbindung(en) modifizierten Füllstoff ausfällt.

Der Feststoffgehalt der eingesetzten Latices beläuft sich im allgemeinen auf 20 bis 25 Gew.-%. Der Feststoffgehalt der Kautschuklösungen beträgt im allgemeinen 3 bis 35 Gew.-%, der der Kautschukemulsionen im allgemeinen 5 bis 30 Gew.-%.

Für die Aufarbeitung von Kautschukpulvern mit Füllstoffgehalten ≥100 phr ist es vorteilhaft, vor der Phasentrennung den pH-Wert bis auf 2,5 abzusenken. Dazu wird zweckmäßigerweise eine Säure aus der vorher genannten Gruppe der Säuren verwendet.

Das erfindungsgemäße Verfahren kann sowohl diskontinuierlich als auch kontinuierlich durchgeführt werden.

Das ausgefällte Kautschukpulver wird vorteilhaft mit Hilfe einer Zentrifuge abgetrennt und dann auf einen Restwassergehalt von im allgemeinen ≤1 % getrocknet, insbesondere in einem Wirbelbettrockner.

Die erfindungsgemäßen Kautschukpulver werden unter Verwendung einer oder mehrerer Organosiliciumverbindungen der allgemeinen Formel

**[**R¹ ₙ- (RO)₃₋ₙ Si-(Alk)ₘ -(Ar)ₚ**]** _{q} **[**B**]** (I),

R¹ ₙ(RO)₃₋ₙSi-(Alk) (II),

oder

R¹ ₙ (RO)₃₋ₙSi-(Alkenyl) (III)

hergestellt,
in denen bedeuten
- B:: -SCN, -SH, -Cl, -NH₂ (wenn q = 1) oder -Sx- (wenn q = 2)
- R und R¹:: eine Alkylgruppe mit 1 bis 4 Kohlenstoffatomen, verzweigt oder nicht verzweigt, den Phenylrest, wobei alle Rest R und R¹ jeweils die gleiche oder eine verschiedene Bedeutung haben können, bevorzugt eine Alkylgruppe,
- R:: eine C₁-C₄-Alkyl, -C₁-C₄-Alkoxygruppe, verzweigt oder nicht verzweigt,
- n:: 0; 1 oder 2,
- Alk:: einen zweiwertigen geraden oder verzweigten Kohlenstoffrest mit 1 bis 6 Kohlenstoffatomen,
- m:: 0 oder 1
- Ar:: einen Arylenrest mit 6 bis 12 C-Atomen
- p:: 0 oder 1 mit der Maßgabe, daß p und m nicht gleichzeitig 0 bedeuten,
- x:: eine Zahl von 2 bis 8,
- Alkyl:: einen einwertigen geraden oder verzweigten ungesättigten Kohlenwasserstoffrest mit 1 bis 20 Kohlenstoffatomen, bevorzugt 2 bis 8 Kohlenstoffatomen,
- Alkenyl:: einen einwertigen geraden oder verzweigten ungesättigten Kohlenwasserstoffrest mit 2 bis 20 Kohlenstoffatomen, bevorzugt 2 bis 8 Kohlenstoffatomen.

Diese Verbindungen werden, wenn sie wasserlöslich sind, im allgemeinen in Form von Lösungen oder ansonsten in Form von Emulsionen eingesetzt, wobei die Emulsionen auch in Gegenwart der Kieselsäuresuspension gebildet werden können.

Man stellt die Emulsion oder Lösung bevorzugt bei Raumtemperatur her. Es sind aber auch Temperaturen von 10 bis 60 ° C geeignet.

Die Konzentration der Organosiliciumverbindung(en) in der Suspension beläuft sich auf 0,5 bis 20 Gew.-%, bevorzugt 5 bis 12 Gew.-%, bezogen auf die Gesamtmenge des eingesetzten Füllstoffs.
Der pH-Wert der Emulsion oder Lösung liegt ebenso wie der pH-Wert der Füllstoffsuspension nach dem Zumischen der Emulsion im schwach sauren oder schwach alkalischen, bevorzugt aber bei einem pH-Wert von etwa 7.

Unter dem verwendeten Begriff wasserunlöslich ist zu verstehen:

Nach dem Vermischen der Organosilanverbindung (ohne oberflächenaktive Substanz) mit der Suspension des Füllstoffs bildet sich um die Füllstoffteilchen herum im gewünschten pH- und Konzentrationsbereich keine klare Lösung. Es bleiben vielmehr die getrennten Phasen, die aus Wasser, Feststoff und Organosiliciumverbindung(en) bestehen. Die oligosulfidischen Organosilane gemäß der oben angegebenen allgemeinen Formel I sind an sich bekannt und können nach bekannten Verfahren hergestellt werden. Beispiele für vorzugsweise eingesetzten Organosilane sind die z. B. nach der BE-PS 787 691 herstellbaren Bis(trialkoxysilyl-alkyl)oligosulfide wie Bis-(trimethoxy-, triäthoxy-, -trimethoxy- äthoxy-, -tripropoxy-, -tributoxy-, -tri-i-propoxy- und -tri-i-butoxy-silylmethyl)-oligosulfide und zwar insbesondere die Di-, Tri-, Tetra-, Penta-, Hexasulfide usw., weiterhin Bis-(2-trimethoxy-, -triäthoxy-, -trimethoxyäthoxy-, -tripropoxy- und -tri-n-und -i-butoxy-äthyl)-oligosulfide und zwar insbesondere die Di-, Tri-, Tetra-, Penta-, Hexasulfide usw., ferner die Bis-(3-trimethoxy-, -triäthoxy-, -trimethoxyäthoxy-, -tripropoxy-, -tri-n-butoxy- und tri-i-butoxysilyl-propyl)-oligosulfide und zwar wiederum die Di-, Tri-, Tetrasulfide usw. bis zu Octasulfiden, des weiteren die entsprechenden Bis-(3-trialkoxysilylisobutyl)-oligosulfide, die entsprechen Bis-(4-trialkoxysilylbutyl)-oligosulfide. Von diesen ausgewählten, relativ einfach aufgebauten Organosilanen der allgemeinen Formel I werden wiederum bevorzugt die Bis-(3-trimethoxy-, -triäthoxy- und tripropoxysilylpropyl)oligosulfide, und zwar die Di-, Tri-, Tetra- und Pentasulfide, insbesondere die Triäthoxyverbindungen mit 2, 3 oder 4 Schwefelatomen und deren Mischungen. Alk bedeutet in der allgemeinen Formel I einen zweiwertigen, geraden oder verzweigten Kohlenwasserstoffrest, vorzugsweise einen gesättigten Alkylenrest mit gerader Kohlenstoffkette mit 1 bis 4 Kohlenstoffatomen.

Speziell geeignet sind auch die Silane mit der folgenden Strukturformel 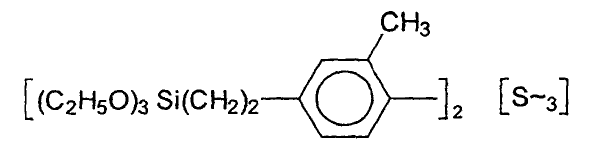 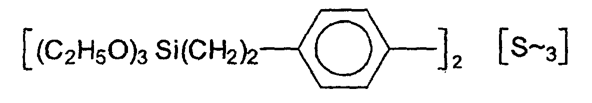 und deren Methoxianaloge, herstellbar nach der DE-AS 25 58191. Diese Verbindungen sind nicht wasserlöslich.

Als oberflächenaktive Substanzen finden in diesem Fall bevorzugt nichtionogene, kationische und anionische Tenside Verwendung. Ihre Konzentration in der Emulsion beträgt 1 bis 15 Gew.-%, bevorzugt 2 bis 10 Gew.-%, bezogen auf die Menge an Organosilanverbindungen

Beispiele für derartige Tenside sind Alkylphenolpolyglycolether, Alkylpolyglycolether,Polyglycole, Alkyltrimethylammoniumsalze, Dialkyldimtheylammoniumsalze, Alkylbenzyltrimethylammomiumsalze, Alkylbenzolsulfonate, Alkylhydrogensulfate, Alkylsulfate.

Die zu modifizierenden natürlichen oder gefällten Füllstoffe, auch als Gemisch von zwei oder mehreren dieser Füllstoffe, sind an sich in der Kautschuktechnologie bekannte Füllstoffe. Wesentliche Voraussetzung für ihre Eignung ist das Vorhandensein von OH-Gruppen an der Oberfläche der Füllstoffteilchen, die mit den Alkoxygruppen der Organosiliciumverbindungen reagieren können. Es handelt sich um oxidische und silikatische Füllstoffe, die mit Kautschuken veträglich sind, und die für diese Verwendung notwendige und bekannte Feinteiligkeit aufweisen.

Als natürliche Silikate sind besonders Kaoline oder Clays geeignet. Aber auch Kieselgur oder Diatomeenerde können eingesetzt werden

Als oxidische Füllstoffe sind beispielhaft zu nennen Aluminiumoxid, Aluminiumhydroxid oder -trihydrat und Titandioxid.

"Modifizierte Füllstoffe" bedeutet in diesem Zusammenhang, daß die Organosilanverbindungen entweder durch chemische Umsetzung (OH-Gruppen) oder adsorptiv an die Oberfläche gebunden sind.

Die adsorptiv gebundenen Gruppen werden durch den Trocknungsschritt in chemisch gebundene umgewandelt.

Die Emulsion wird in derartigen Mengen mit der Füllstoffsuspension vermischt, daß die Konzentration der Organosiliciumverbindung 0,5 bis 20 Gew.-%, bevorzugt 5 bis 12 Gew.-%, bezogen auf die Füllstoffmenge beträgt.Die modifizierten Füllstoffe enthalten 0,5 bis 20 Gew.-%, bevorzugt 0,5 bis 12 Gew.-%, der Organosiliciumverbindungen, bezogen auf den trockenen Füllstoff.

Sie sind besonders geeignet zur Verwendung in vulkanisierund formbaren Kautschukmischungen.
Man setzt für das erfindungsgemäße Verfahren vorteilhaft einen salzfrei gewaschenen Filterkuchen aus der Kieselsäurefällung ein.
Geeignet sind auch Suspensionen, wie man sie bei der Aufarbeitung von natürlichen Füllstoffen wie Clays erhält.

Man spart so gegenüber dem Stand der Technik einen energieaufwendigen Trocknungsschritt.

Die eingesetzten Kieselsäuren sind aus dem Kautschuksektor bekannt.

Sie besitzen im allgemeinen eine nach der bekannten BET-Methode bestimmte N₂-Oberfläche von 35 bis 700 m²/g, eine CTAB-Oberfläche von 30 bis 500 m²/g, eine DBP-Zahl von 150 bis 400 ml/100g.
Das erfindungsgemäße Produkt enthält diese Kieselsäuren in einer Menge von 5 bis 250 Teilen, insbesondere 20 bis 100 Teilen, bezogen auf 100 Teile Kautschuk.

Handelt es sich um weiße Naturfüllstoffe, wie Clays oder Kieselkreiden mit einer N₂-Oberfläche von 2 bis 35 m²/g setzt man diesen in einer Menge von 5 bis 350 Teilen, bezogen auf 100 Teile Kautschuk, ein.

Herstellbar sind auch füllstoffhaltige Kautschukpulver, die Kieselsäuren und Ruß im Gemisch enthalten.
Die Gesamtmenge an Füllstoff überschreitet 250 phr aber nicht.

Besonders geeignet sind Ruße, wie sie allgemein in der Kautschukverarbeitung eingesetzt werden.

Dazu gehören Furnaceruße, Gas- und Flammruße mit einer Jodadsorptionszahl 5 bis 1000 m²/g, einer CTAB-Zahl von 15 bis 600 m²/g, einer DBP-Adsorption von 30 bis 400 ml/100 g und einer 24 M4 DBP-Zahl von 50 bis 370 ml/100 g in einer Menge von 5 bis 100 Teilen, insbesondere 20 bis 100 Teilen, auf 100 Teile Kautschuk.

Als Kautschuktypen einsetzbar und als wässrige Emulsionen darstellbar haben sich folgende Spezies gezeigt, einzeln oder im Gemisch miteinander:
Naturkautschuk, Emulsions-SBR mit einem Styrolanteil von 10 bis 50 %, Butyl-Acrylnitril-Kautschuk.
Butylkautschuke, Terpolymere aus Ethylen, Propylen (EPM) und nicht konjugierte Diene (EPDM), Butadienkautschuke, SBR, hergestellt nach dem Lösungspolymerisationsverfahren, mit Styrolgehalten von 10 bis 25 %, sowie Gehalten an 1,2-Vinylbestandteilen von 20 bis 55 % und Isoprenkautschuke, insbesondere 3,4-Polyisopren.

Bei nach Lösungsmittelverfahren hergestellten Polymerisaten sind besondere Vorsichtsmaßnahmen wegen des Lösungsmittelgehalts zu treffen.

Neben den genannten Kautschuken kommen folgende Elastomere, einzeln oder im Gemisch, in Frage:
Carboxylkautschuke, Epoxidkautschuke, Trans-Polypentenamer, halogenierte Butylkautschuke, Kautschuke aus 2-Chlor-Butadien, Ethylen-Vinylacetat-Copolymere, Epichlorhydrine, gegebenfalls auch chemisch modifizierter Naturkautschuk, wie z. B. epoxidierte Typen.

Die erfindungsgemäßen Kautschukpulver enthalten neben den bereits genannten Füllstoffen gegebenfalls bekannte Verarbeitungs- oder Vulkanisationshilfsmittel wie Zinkoxid, Zinkstearat, Stearinsäure, Polyalkohole, Polyamine, Weichmacher, Alterungsschutzmittel gegen Wärme, Licht oder Sauerstoff und Ozon, Verstärkerharze, Flammschutzmittel wie z. B. Al(OH)₃ und Mg(OH)₂, Pigmente,verschiedene Vernetzungschemikalien und gegebenfalls Schwefel in den gummitechnisch üblichen Konzentrationen.

Es gelingt erfindungsgemäß, feinteiliges, mit Organosilicumverbindungen modifizierte Kieselsäure enthaltendes Kautschukpulver herzustellen, das rieselfähig ist und auch nach mechanischer Beanspruchung (z. B. Fördern, Verpacken) rieselfähig bleibt.
Aufgrund seiner Feinteiligkeit sind keine Mahl- oder sonstige Zerkleinerungsmaßnahmen notwendig, um feinteilige Dispersionen zu erhalten.

Diese führen dann zu den feinteiligen Kautschukpulvern, die sich leicht verarbeiten lassen, und zu Vulkanisaten mit verbesserten Eigenschaften.

In den nachfolgenden Beispielen werden die Durchführbarkeit und die Vorteile der vorliegenden Erfindung erläutert, ohne daß sie auf diese demonstrierten Maßnahmen eingeschränkt sind.

### Eingesetzte Rohstoffe bei der Herstellung

| | |
|---|---|
| E - SBR | Emulsions - Styrolbutadienlatex mit 23,5 % Styrolgehalt (BSL) |
| | |
| Si 69 | Bis(triethoxysilylpropyl)tetrasulfan (Degussa AG) |
| | |
| Si 75 | Bis(triethoxysilylpropyl)disulfan (Degussa AG) |
| | |
| Ultrasil VN3, Ultrasil VN3 Filterkuchen | gefällte Kieselsäure mit einer N₂-Oberfläche (BET) von 175 m²/g (Degussa AG) getrocknet oder als Filterkuchen, gegebenenfalls granuliert (gran) |
| | |
| Ultrasil 7000, Ultrasil 7000 Filterkuchen | gefällte Kieselsäure mit einer N₂-Oberfläche (BET)von 175 m²/g und verbesserten Dispergiereigenschaften (Degussa AG) getrocknet oder als Filterkuchen, gegebenenfalls granuliert (gran) |
| | |
| Marlipal 1618/25 | Emulgator : Fettalkoholpolyethylen - glykolether (Hüls AG) |

### Beispiel I

### Herstellung von Pulverkautschuk auf Basis E-SBR, Ultrasil 7000 und Si 69

Unter Rühren wird eine stabile Suspension aus 14,3 kg Ultrasil 7000, 1,58 kg Si 69 ( entspricht 11,3 % bezogen auf die Kieselsäure ), 142g Marlipal 1618/25 (entspricht 1 bez. auf die Kieselsäure ) in 255 L Wasser hergestellt und anschließend im Verhältnis 5 : 1 aufgeteilt.

Der größere Anteil der Suspension wird mit 94,3 L einer 21,0 %igen E-SBR Latexemulsion unter heftigem Rühren vermischt und anschließend durch Zugabe einer ca. 10%igen Al₂(SO₄)₃ Lösung auf einen pH - Wert von 5,0 abgesenkt. Diesem ersten Fällschritt folgt die Zugabe des wie oben hergestellten zweiten Teils der Suspension mit anschließender Absenkung des pH - Wertes auf einen Endpunkt von 3,7.

Nach dem Fällprozeß erfolgt eine mechanische Abtrennung des größten Teils des Wassers, gefolgt von einem Trocknungsschritt auf eine Restfeuchte von < 1 %. Das pulverförmige Fertigprodukt (EPB 1) enthält 100 Teile E-SBR und 77 Teile Ultrasil 7000 / Si 69 ( 11,3 % ), bestimmt mittels Thermogravimetrischer Analyse ( TGA ).

### Beispiel II

### Herstellung von Pulverkautschuk auf Basis E-SBR, Ultrasil 7000 Filterkuchen und Si 69

Unter Rühren wird eine stabile Suspension aus 59,0 kg Ultrasil 7000 Filterkuchen, 1,60 kg Si 69 (entspricht 11,3 % bezogen auf die Kieselsäure ), 140g Marlipal 1618/25 (entspricht 1 % bez. auf die Kieselsäure) in 189 L Wasser hergestellt und anschließend im Verhältnis 5 : 1 aufgeteilt.

Der größere Anteil der Suspension wird mit 95,7 L einer 20,5 %igen E-SBR Latexemulsion unter heftigem Rühren vermischt und anschließend durch Zugabe einer ca. 10 %igen Al₂(SO₄)₃ Lösung auf einen pH - Wert von 4,9 abgesenkt. Diesem ersten Fällschritt folgt die Zugabe des wie oben hergestellten zweiten Teils der Suspension mit anschließender Absenkung des pH - Wertes auf 3,4.

Nach dem Fällprozeß erfolgt eine mechanische Abtrennung des größten Teils des Wassers, gefolgt von einem Trocknungsschritt auf eine Restfeuchte von < 1 %. Das pulverförmige Fertigprodukt (EPB 2) enthält 100 Teile E-SBR und 83 Teile Ultrasil 7000 (aus Filterkuchen) / Si 69 (11,3 %), (TGA - Bestimmung).

### Beispiel III

### Herstellung von Pulverkautschuk auf Basis E-SBR, Ultrasil VN3 und Si 69

Unter Rühren wird eine stabile Suspension aus 13,9 kg Ultrasil VN3, 1,55 kg Si 69 (entspricht 11,3 % bezogen auf die Kieselsäure), 137g Marlipal 1618/15 (entspricht 1 % bez. auf die Kieselsäure) in 267 L Wasser hergestellt und anschließend im Verhältnis 5 : 1 aufgeteilt.

Der größere Anteil der Suspension wird mit 94,7 L einer 20,9 %igen E-SBR Latexemulsion unter heftigem Rühren vermischt und anschließend durch Zugabe einer ca. 10 %igen Al₂(SO₄)₃ Lösung auf einen pH - Wert von 5,2 abgesenkt. Diesem ersten Fällschritt folgt die Zugabe des wie oben hergestellten zweiten Teils der Suspension mit anschließender Absenkung des pH - Wertes auf 3,5.

Nach dem Fällprozeß erfolgt eine mechanische Abtrennung des größten Teils des Wassers, gefolgt von einem Trocknungsschritt auf eine Restfeuchte von < 1 % . Das pulverförmige Fertigprodukt (EPB 3) enthält 100 Teile E-SBR und 72 Teile Ultrasil VN3 / Si 69 (11,3 %), (TGA - Bestimmung).

### Beispiel IV

### Herstellung von Pulverkautschuk auf Basis E-SBR, Ultrasil 7000 und Si 75

Unter Rühren wird eine stabile Suspension aus 14,6 kg Ultrasil 7000, 1,59 kg Si 75 (entspricht 11,3 % bezogen auf die Kieselsäure ), 142g Marlipal 1618/15 (entspricht 1 % bez. auf die Kieselsäure ) in 258 L Wasser hergestellt und anschließend im Verhältnis 5 : 1 aufgeteilt.

Der größere Anteil der Suspension wird mit 93,8 L einer 21,5 %igen E-SBR Latexemulsion unter heftigem Rühren vermischt und anschließend durch Zugabe einer ca. 10 %igen Al₂(SO₄)₃ Lösung auf einen pH - Wert von 5,1 abgesenkt. Diesem ersten Fällschritt folgt die Zugabe des zweiten Teils der Suspension (Sättigungsmittel)mit anschließender Absenkung des pH - Wertes auf 3,3.

Nach dem Fällprozeß erfolgt eine mechanische Abtrennung des größten Teils des Wassers, gefolgt von einem Trocknungsschritt auf eine Restfeuchte von < 1 %. Das pulverförmige Fertigprodukt (EPB 4) enthält 100 Teile E - SBR und 76 Teile Ultrasil 7000 / Si 75 (11,3 %), (TGA - Bestimmung).

### Beispiel V

### Herstellung von Pulverkautschuk auf Basis E-SBR, Ultrasil 7000 Filterkuchen Si 75

Unter Rühren wird eine stabile Suspension aus 61,0 kg Ultrasil 7000 Filterkuchen, 1,63 kg Si 75 (entspricht 11,3 % bezogen auf die Kieselsäure), 140 g Marlipal 1618/25 (entspricht 1 % bez. auf die Kieselsäure) in 195 l Wasser hergestellt und anschließend im Verhältnis 5 : 1 aufgeteilt.

Der größere Teil der Suspension wird mit 96,2 l einer 20,5 %igen E-SBR Latexemulsion unter hefigem Rühren vermischt und anschließend durch Zugabe einer ca. 10 %igen Al₂(SO₄)₃-Lösung auf eine pH-Wert von 4,8 abgesenkt. Diesem ersten Fällschritt folgt die Zugabe des zweiten Teils der Suspension (Sättigungsmittel) mit anschließender Absenkung des pH-Wertes auf 3,5.

Nach dem Fällprozeß erfolgt eine mechanische Abtrennung des größten Teils des Wassers, gefolgt von einem Trocknüngsschritt auf eine Restfeuchte von <1 %. Das pulverförmige Fertigprodukt (EPB 5) enthält 100 Teile E-SBR und 80 Teile Ultrasil 7000(aus Filterkuchen) / Si 75 (11,3 %), (TGA-Bestimmung).

In der gummitechnischen Anwendung wurden folgende Produkte eingesetzt:

### Chemikalien

| | |
|---|---|
| SBR 1500 | Styrol-Butadien Kautschuk mit 23,5 % Styrolgehalt |
| | |
| Naftolen ZD | arom. Mineralölweichmacher |
| | |
| EPB 1 | Pulverkautschuk, bestehend aus 100 Teilen E-SBR 1500, 77 Teilen Ultrasil 7000 /Si69 |
| | |
| EPB 2 | Pulverkautschuk, bestehend aus 100 Teilen E-SBR 1500, 83 Teilen Ultrasil 7000 (ausFilterkuchen) /Si69 |
| | |
| EPB 3 | Pulverkautschuk, bestehend aus 100 Teilen E-SBR 1500, 72 Teilen Ultrasil VN3 /Si69 |
| | |
| EPB 4 | Pulverkautschuk, bestehend aus 100 Teilen E-SBR 1500, 76 Teilen Ultrasil 7000 /Si75 |
| | |
| EPB 5 | Pulverkautschuk, bestehend aus 100 Teilen E-SBR, 80 Teilen Ultrasil 7000 (aus Filterkuchen) / Si75 |
| | |
| 6 PPD | N-(1,3-Dimethylbuthyl)-N-phenyl-p-phenylendiamin |
| | |
| CBS | Benzothiazyl-2-cyclohexylsulfenamid |
| | |
| DPG | Diphenylguanidin |

Folgende gummitechnische Prüfmethoden wurden angewandt:

| | |
|---|---|
| Mooney - Viskosität | DIN 53 523/3 |
| Zugversuch am Stab | DIN 53 504 |
| Shore Härte | DIN 53 505 |
| Weiterreißwiderstand | ASTM D 624 |
| Abrieb | DIN 53 516 |
| Dispersion (Philips) | ISO/DIS 11 345 |
| Dispersion (Rauhigkeit) | DIN 4788 |
| Bruchdehnung | DIN 53504 |
| Bruchenergie | DIN 53504 |

### Beispiel A

### Vergleich des Gummitechnischen Wertebildes des erfindungsgemäßen Produktes(Herstellungsbeispiel I) gegen eine Standardmischung

### a) Rezeptur

| Mischung | 1 [phr] | 2 [phr] |
|---|---|---|
| SBR 1500 | 100 | - |
| EPB 1 | - | 177 |
| Ultrasil 7000 Gran | 70 | - |
| Si 69 | 9 | - |
| Naftolen ZD | 25 | 25 |
| ZnO | 3 | 3 |
| Stearinsäure | 2 | 2 |
| 6 PPD | 2 | 2 |
| Wachs | 1 | 1 |
| | | |
| CBS | 1,7 | 1,7 |
| DPG | 2 | 2 |
| Schwefel | 1,5 | 1,5 |

### b) Mischverfahren

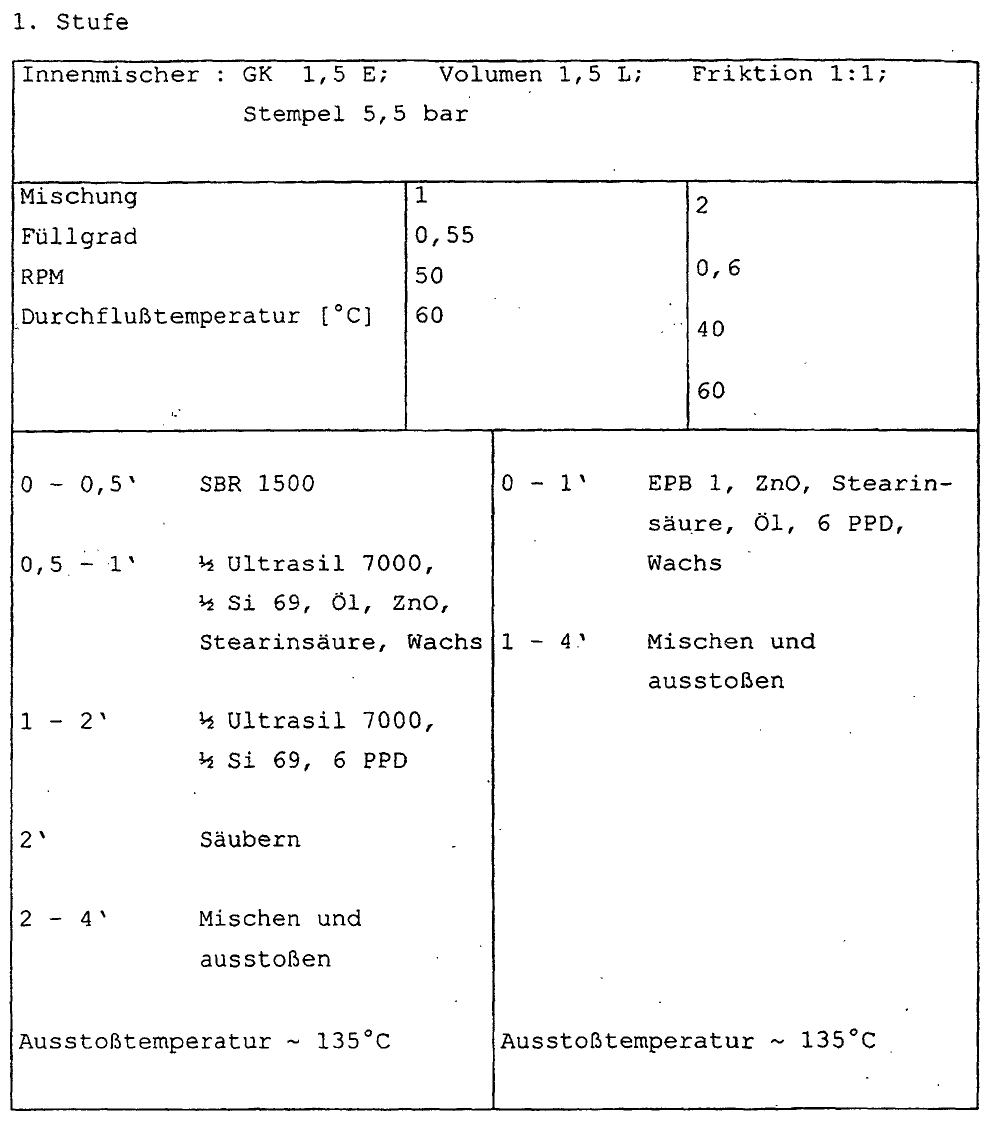

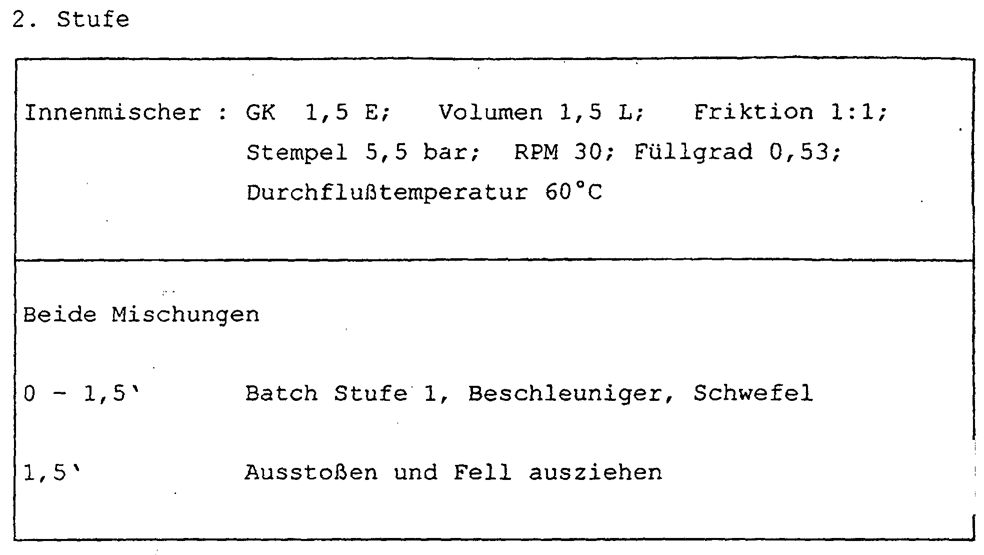

### c) Gummitechnische Daten

| Mischungsnummer | 1 | 2 |
|---|---|---|
| | | |
| ML 1+4 | 45 | 51 |
| | | |
| Zugfestigkeit [MPa] | 21,4 | 25,0 |
| Bruchdehnung [%] | 450 | 600 |
| Bruchenergie [J] | 20,3 | 30,8 |
| Shore-A-Härte | 71 | 69 |
| Weiterreißwiderstand ASTM DIE C [N/mm] | 50 | 57 |
| | | |
| Abrieb [mm³] | 82 | 69 |
| | | |
| Dispersion (Philips) | 7 | 8 |
| Dispersion (Rauhigkeit) Rauhigkeitsfaktor Pc² Pa | 613 | 36 |
| | | |

Der Pulverkautschuk aus E-SBR-Latex, Ultrasil 7000 und Si 69 zeichnet sich gegenüber der konventionellen Mischweise durch höhere Festigkeitswerte, einen günstigeren Abrieb und eine deutlich verbesserte Dispersion aus.

### Beispiel B

### Vergleich des gummitechnischen Wertebildes des erfindungsgemäßen Produktes EPB 2 (E-SBR / Ultrasil VN3/Si69), EPB 3 (E-SBR / Ultrasil VN3 Filterkuchen/Si69)

### a) Rezeptur

| Mischung | 1 [phr] | 2 [phr] |
|---|---|---|
| EPB 2 | 172 | - |
| EPB 3 | - | 172 |
| Naftolen ZD | 25 | 25 |
| ZnO | 3 | 3 |
| Stearinsäure | 2 | 2 |
| 6 PPD | 2 | 2 |
| Wachs | 1 | 1 |
| CBS | 1,7 | 1,7 |
| DPG | 2 | 2 |
| Schwefel | 2,2 | 2, |

### b) Mischverfahren

### 1. Stufe

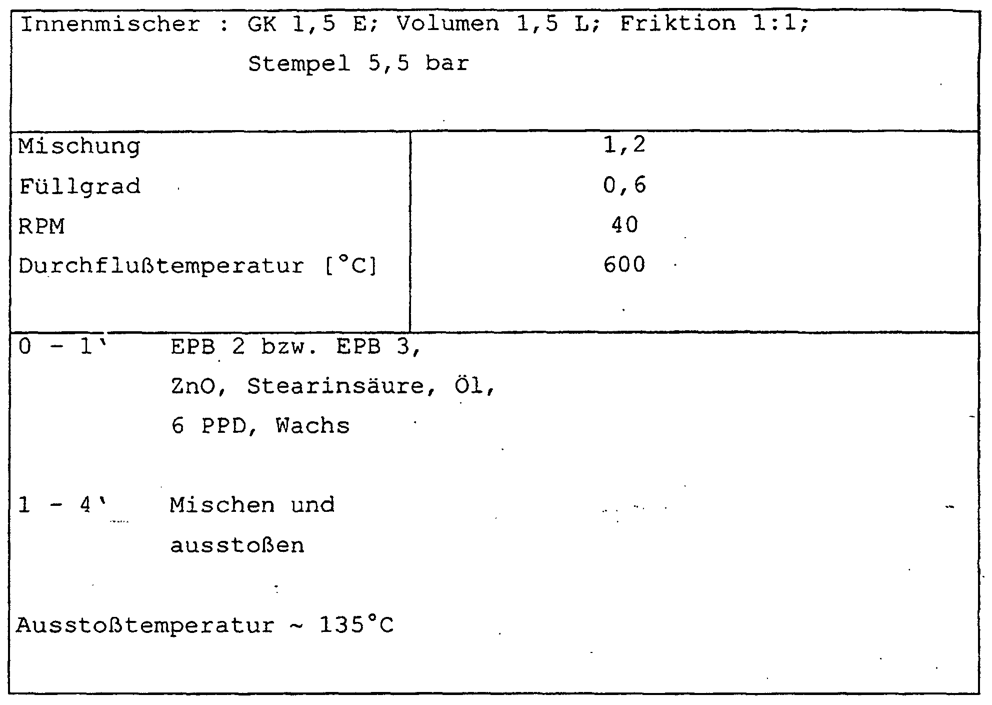

### 2. Stufe

| | |
|---|---|
| Innenmischer : | GK 1,5 E; Volumen 1,5 L; Friktion 1:1; Stempel 5,5 bar; RPM 30; Füllgrad 0,53; Durchflußtemperatur 60°C |
| Beide Mischungen | |
| 0 - 1,5' | Batch Stufe 1, Beschleuniger, Schwefel |
| 1,5' | Ausstoßen und Fell ausziehen |

### c) Gummitechnische Daten

| Mischungsnummer | 1 | 2 |
|---|---|---|
| ML 1+4 | 51 | 56 |
| Zugfestigkeit [MPa] | 19,7 | 21,0 |
| Modul 300 % [MPa] | 6,4 | 6,4 |
| Bruchdehnung [%] | 630 | 650 |
| Bruchenergie [J] | 163 | 180 |
| Shore-Härte | 67 | 69 |
| Din-Abrieb [mm³] | 99 | 88 |
| Dispersion (Philips) | 5 | 8 |
| Dispersion (Rauhigkeit) Rauhigkeitsfaktor Pc² Pa | 3000 | 108 |

Der Pulverkautschuk aus E-SBR-Latex, Ultrasil VN3 Filterkuchen und Si 69 (EPB 3) zeichnet sich gegenüber dem Ultrasil VN 3 (EPD 2) durch höhere Festigkeitswerte, einen besseren Abrieb und durch eine exzellente Dispersion im Compound aus.

### Beispiel C

### Vergleich zwischen EPB 4 (E-SBR / Ultrasil VN3 Filterkuchen / Si69) und EPB 5 (E-SBR / Ultrasil 7000 Filterkuchen / Si69)

### a) Rezeptur

| Mischung | 1 [phr] | 2 [phr] |
|---|---|---|
| EPB 4 | 176 | - |
| EPB 5 | - | 180 |
| Naftolen ZD | 25 | 25 |
| ZnO RS | 3 | 3 |
| Stearinsäure | 2 | 2 |
| 6 PPD | 2 | 2 |
| Wachs | 1 | 1 |
| CBS | 1,7 | 1,7 |
| DPG | 2 | 2 |
| Schwefel | 1,5 | 1,5 |

### b) Mischverfahren

### 1. Stufe

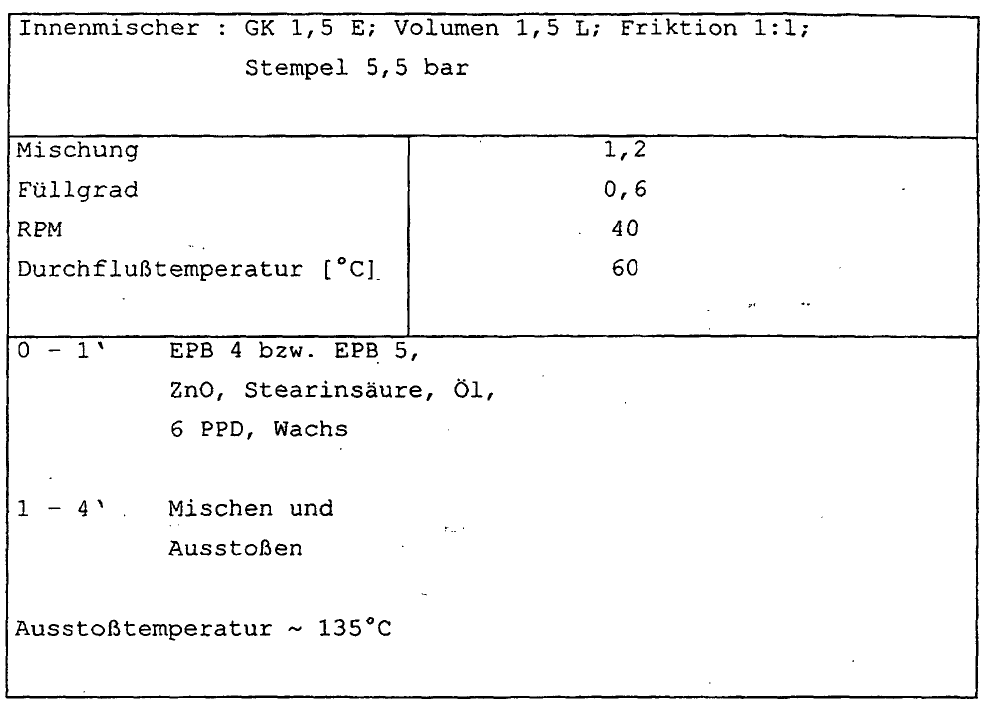

### 2. Stufe

| | |
|---|---|
| Innenmischer : | GK 1,5 E; Volumen 1,5 L; Friktion 1:1; Stempel 5,5 bar; RPM 30; Füllgrad 0,53; Durchflußtemperatur 60°C |
| Beide Mischungen | |
| 0 - 1,5' | Batch Stufe 1, Beschleuniger, Schwefel |
| 1,5' | Ausstoßen und Fell ausziehen |

### c) Gummitechnische Daten

| Mischungsnummer | 1 | 2 |
|---|---|---|
| Zugfestigkeit [MPa] | 18,2 | 20,3 |
| Modul 300 % [MPa] | 6,7 | 8,9 |
| Bruchenergie [J] | 138 | 149 |
| Shore-Härte | 70 | 72 |
| DIN-Abrieb [mm³] | 97 | 81 |
| Dispersion (Philips) | 8 | 9 |
| Dispersion (Rauhigkeit) Rauhigkeitsfaktor Pc² Pa | 90 | 16 |

Das Produkt EPB 5 mit Ultrasil 7000 Filterkuchen / Si 75 zeichnet sich gegenüber dem Ultrasil VN3 Filterkuchen (EPB 4) durch noch bessere Festigkeitswerte, eine weitere Erhöhung des Abriebwiderstands und eine bessere Dispersion aus.

## Patentansprüche

1. Verfahren zur Herstellung feinteiliger Kautschuke (Kautschukpulver) durch Ausfällen aus wasserhaltigen Mischungen, die Füllstoff in Form von Suspensionen, wasserlösliche Salze eines Metalls der Gruppen IIa, IIb, IIIa und VIII des periodischen Systems der Elemente und einen Kautschuklatex (Polymerlatex), wässrige Emulsionen eines Kautschuks oder Kautschuklösung enthalten,
**dadurch gekennzeichnet,**
**daß** man
a) eine oder mehrere Organosiliciumverbindung(en), die mindestens eine Alkoxygruppe enthalten, in Wasser gelöst oder gegebenenfalls in Anwesenheit einer oberflächenaktiven Substanz emulgiert, oder die genannten Verbindungen direkt, gegebenenfalls mit einer oberflächenaktiven Substanz mit der wässrigen Suspension eines oxidischen oder silikatischen feinteiligen Füllstoffs oder einer Mischung dieser Füllstoffe mit einem mittleren Teilchendurchmesser < 10 µm bei einer Temperatur von 10 bis 60 °C, bevorzugt bei Raumtemperatur, unter Rühren vermischt, wobei man die für die Einarbeitung in den Kautschuk vorgesehene Menge dieses Gemisches, bezogen auf den Füllstoffanteil in zwei Partien aufteilt, und
b) eine erste Partie mit dem Polymerlatex, der Polymeremulsion bzw. der Polymerlösung vermischt, den pH-Wert dieser Mischung mit einer Säure, insbesondere einer Lewissäure, auf einen pH-Wert von 6,0 bis 4,5 absenkt (erste Partie, erste Stufe),
c) den restlichen Anteil (zweite Partie, Splittinganteil) unter weiterer Absenkung des pH-Wertes auf 4,5 bis 2,6 insbesondere ca. 3,2 zusetzt (zweite Stufe), sodaß der in der Mischung befindliche Kautschuk zusammen mit dem durch Organosiliciumverbindung(en) modifizierten Füllstoff ausfällt
d) den ausgefallenen Feststoff mit an sich bekannten Maßnahmen abtrennt,
e) ihn bevorzugt wäscht, um den pH-Wert auf einen für die weitere Verarbeitung verträglicheren Wert von ca. 6 bis 7 einzustellen, und
f) den füllstoffhaltigen Kautschuk trocknet.

2. Verfahren gemäß Anspruch 1,
**dadurch gekennzeichnet,**
**daß** man eine oder mehrere Organosiliciumverbindungen der allgemeinen Formel
**[**R¹ ₙ-(RO)₃₋ₙ Si-(Alk)ₘ -(Ar)ₚ**]** _{q}**[**B**]** (I),
R¹ ₙ(RO)₃₋ₙ Si-(Alk) (II),
oder
R¹ ₙ(RO)₃₋ₙ Si-(Alkenyl) (III)
einsetzt,
in denen bedeuten
B: -SCN, -SH, -Cl, -NH₂ (wenn q = 1) oder -Sx- (wenn q = 2)
R und R¹: eine Alkylgruppe mit 1 bis 4 Kohlenstoffatomen,
verzweigt oder nicht verzweigt, den Phenylrest,
wobei alle Rest R und R¹ jeweils die gleiche oder eine verschiedene Bedeutung haben können,
bevorzugt eine Alkylgruppe,
R: eine C₁-C₄-Alkyl, -C₁-C₄-Alkoxygruppe, verzweigt oder nicht verzweigt,
n: 0; 1 oder 2,
Alk: einen zweiwertigen geraden oder verzweigten Kohlenstoffrest mit 1 bis 6 Kohlenstoffatomen,
m: 0 oder 1
Ar: einen Arylenrest mit 6 bis 12 C-Atomen
p: 0 oder 1 mit der Maßgabe, daß p und m nicht gleichzeitig 0 bedeuten,
x: eine Zahl von 2 bis 8,
Alkyl: einen einwertigen geraden oder verzweigten ungesättigten Kohlenwasserstoffrest mit 1 bis 20 Kohlenstoffatomen, bevorzugt 2 bis 8 Kohlenstoffatomen,
Alkenyl: einen einwertigen geraden oder verzweigten ungesättigten Kohlenwasserstoffrest mit 2 bis 20 Kohlenstoffatomen, bevorzugt 2 bis 8 Kohlenstoffatomen.

3. Verfahren gemäß den Ansprüchen 1 oder 2,
**dadurch gekennzeichnet,**
**daß** man als oberflächenaktive Substanzen nichtionogene, kationische oder anionische Tenside einsetzt.

4. Verfahren gemäß Anspruch 3,
**dadurch gekennzeichnet,**
**daß** man die Tenside in einer Menge von 1 bis 15 Gew.-%, insbesondere 2 bis 10 Gew.-%, bezogen auf die Menge am Organosilanverbindungen in der Emulsion, einsetzt.

5. Verfahren gemäß einem oder mehreren der vorhergehenden Ansprüche,
**dadurch gekennzeichnet,**
**daß** die Konzentration der Organosiliciumverbindung in der Suspension 0,3 bis 15 Gew.-%, bezogen auf den Füllstoff (atro), beträgt.

6. Verfahren gemäß Anspruch 1,
**dadurch gekennzeichnet,**
**daß** man das Kautschukpulver in Gegenwart einer Säure, bevorzugt einer Lewissäure, insbesondere Al₂ (SO₄)₃, ausfällt.

7. Verfahren gemäß Anspruch 3,
**dadurch gekennzeichnet,**
**daß** man ein Tensid aus der Klasse der Fettalkoholpolyethylenglykolether oder Alkylphenolpolyethylenglykolether einsetzt.

8. Verfahren gemäß einem oder mehreren der vorhergehenden Ansprüche,
**dadurch gekennzeichnet,**
**daß** man in Stufe a) als Füllstoff insbesondere den salzfrei gewaschenen Filterkuchen einer gefällten Kieselsäure einsetzt.

9. Verfahren gemäß einem oder mehreren der vorhergehenden Ansprüche,
**dadurch gekennzeichnet,**
**daß** man den bei der Aufarbeitung natürlicher Füllstoffe, insbesondere Clays, anfallenden Feststoff einsetzt.

10. Verfahren gemäß Anspruch 1,
**dadurch gekennzeichnet,**
**daß** man in der ersten Partie gemäß Schritt b) ≥ 50 Teile des im Kautschukpulver vorgesehenen Füllstoffanteils einsetzt.

11. Verfahren gemäß Anspruch 1,
**dadurch gekennzeichnet,**
**daß** man in der ersten Partie gemäß b) bei einem Anteil von ≥ 80 Teilen Füllstoff phr (per hundred parts of rubber) 90 bis 99 % des im Kautschukpulver vorgesehenen Füllstoffanteils einsetzt

12. Verfahren gemäß einem oder mehreren der vorhergehenden Ansprüche,
**dadurch gekennzeichnet,**
**daß** man der Emulsion/Suspension gemäß dem Schritt a) oder der Mischung gemäß Schritt b) einen gummitechnisch einsetzbaren Ruß in der gewünschten Menge zusetzt.

13. Verfahren gemäß Anspruch 1,
daß man entweder der Emulsion, Suspension oder Lösung gemäß Anspruch 1, Punkt a) oder den gemäß Punkten b) c) erzeugten Gemisch in der Kautschukindustrie übliche Verarbeitungshilfsmittel, Alterungsschutzmittel, Aktivatoren und/oder Vernetzungschemikalien und Schwefel in den üblichen Mengen zusetzt.

14. Feinteilige Kautschuke (Kautschukpulver) hergestellt nach Anspruch 1, die Kautschuk, Füllstoffe und in der Kautschukindustrie übliche Verarbeitungsmittel, Alterungsschutzmittel, Aktivatoren und/oder Vernetzungschemikalien und Schwefel in den üblichen Mengen enthalten.

## Claims

1. A process for producing a finely divided rubber (a rubber powder) by precipitation from an aqueous mixture containing a filler in the form of a suspension, a water-soluble salt of a metal of group IIa, IIb, IIIa or VIII of the periodic table and a rubber latex (polymer latex), an aqueous emulsion of a rubber or a rubber solution,
**characterized in that**
a) one or more organosilicon compounds containing at least one alkoxy group are dissolved in water or if desired emulsified in water in the presence of a surface-active substance, or the compounds mentioned are mixed directly, if desired with a surface-active substance, with the aqueous suspension of a finely divided oxidic or silicatic filler or a mixture of these fillers having an average particle diameter < 10 µm at a temperature in the range from 10 to 60°C and preferably at room temperature with stirring, the amount of this mix envisaged for incorporation in the rubber being subdivided into two lots, based on the filler fraction, and
b) a first lot is mixed with the polymer latex, emulsion or solution, the pH of this mixture is lowered with an acid, especially with a Lewis acid, to a pH in the range from 6.0 to 4.5 (first lot, first stage),
c) the remaining fraction (second lot, splitting fraction) is added with continued lowering of the pH to a value in the range from 4.5 to 2.6 and especially about 3.2 (second stage) so that the rubber present in the mixture precipitates together with the filler modified by one or more organosilicon compounds,
d) the precipitated solid is separated off in a conventional manner,
e) it is preferably washed in order that the pH may be adjusted to a more propitious value for further processing, a value in the range from about 6 to 7, and
f) the filler-containing rubber is dried.

2. A process according to claim 1,
**characterized in that**
it utilizes one or more organosilicon compounds of the general formula
[R¹ ₙ-(RO)₃₋ₙSi-(Alk)ₘ-(Ar)ₚ]_{q}[B] (I),
R¹ ₙ(RO)₃₋ₙSi-(Alk) (II),
or
R¹ ₙ(RO)₃₋ₙSi-(Alkenyl) (III)
where
B is -SCN, -SH, -Cl, -NH₂ (when q = 1) or -Sx- (when q = 2)
R and R¹ are each an alkyl group having 1 to 4 carbon atoms,
branched or unbranched,
phenyl,
all R and R¹ radicals may each be the same or different,
preferably an alkyl group,
R is a C₁-C₄-alkyl, -C₁-C₄-alkoxy group, branched or unbranched,
n is 0, 1 or 2,
Alk is a bivalent straight or branched hydrocarbyl radical having 1 to 6 carbon atoms,
m is 0 or 1,
Ar is an arylene radical having 6 to 12 carbon atoms,
p is 0 or 1 with the proviso that p and m are not 0 at one and the same time,
x is a number from 2 to 8,
Alkyl is a univalent straight or branched unsaturated hydrocarbyl radical having 1 to 20 carbon atoms and preferably 2 to 8 carbon atoms,
Alkenyl is a univalent straight or branched unsaturated hydrocarbyl radical having 2 to 20 carbon atoms and preferably 2 to 8 carbon atoms.

3. A process according to claim 1 or 2,
**characterized in that**
a nonionic surfactant, a cationic surfactant or an anionic surfactant is used as surface-active substance.

4. A process according to claim 3,
**characterized in that**
the surfactant is used in an amount in the range from 1% to 15% by weight and especially in the range from 2% to 10% by weight, based on the amount of organosilane compound in the emulsion.

5. A process according to one or more of the preceding claims,
**characterized in that**
the concentration of organosilicon compound in the suspension is in the range from 0.3% to 15% by weight, based on the filler (bone dry) .

6. A process according to claim 1,
**characterized in that**
the rubber powder is precipitated in the presence of an acid, preferably a Lewis acid, especially Al₂(SO₄)₃.

7. A process according to claim 3,
**characterized in that**
a surfactant from the class of the fatty alcohol polyethylene glycol ethers or alkylphenol polyethylene glycol ethers is used.

8. A process according to one or more of the preceding claims,
**characterized in that**
the filler used in stage a) is in particular the filter cake of a precipitated silica that has been washed salt free.

9. A process according to one or more of the preceding claims,
**characterized in that**
the solid material arising in the course of the work-up of natural fillers, especially clays, is used.

10. A process according to claim 1,
**characterized in that**
the first lot of step b) utilizes ≥ 50 parts of the filler fraction envisaged in the rubber powder.

11. A process according to claim 1,
**characterized in that**
the first lot as per b) utilizes from 90% to 99% of the filler fraction envisaged in the rubber powder when the envisaged filler fraction is ≥ 80 parts of filler per hundred parts of rubber (phr).

12. A process according to one or more of the preceding claims,
**characterized in that**
a rubber grade carbon black is added in the desired amount to the emulsion/suspension of step a) or to the mixture of step b).

13. A process according to claim 1,
**characterized in that**
either the emulsion, suspension or solution as per item a) in claim 1 or the mix generated as per items b) c) of claim 1 is admixed with customary rubber industry processing aids, antioxidants, activators and/or crosslinking chemicals and sulphur in a customary amount.

14. A finely divided rubber (a rubber powder) produced according to claim 1, comprising rubber, a filler and customary rubber industry processing aids, antioxidants, activators and/or crosslinking chemicals and sulphur in a customary amount.

## Revendications

1. Procédé de préparation de caoutchoucs finement divisés (poudre de caoutchouc) par précipitation de mélanges contenant de l'eau, qui contiennent une matière de charge sous forme de suspensions, des sels solubles dans l'eau d'un métal des groupes IIa, IIb, IIIa et VIII du Tableau Périodique des Eléments et un latex de caoutchouc (latex polymère), des émulsions aqueuses d'un caoutchouc ou une solution de caoutchouc,
**caractérisé en ce que**
a) on dissout dans de l'eau un ou plusieurs composés organiques du silicium, qui contiennent au moins un groupe alcoxy, ou éventuellement on les émulsifie en présence d'une substance tensioactive, ou encore on mélange sous agitation les composés mentionnés ci-dessus, directement ou éventuellement avec une substance tensioactive, à la solution aqueuse d'une matière de charge finement divisée de type oxyde ou silicate ou un mélange de ces matières de charge ayant une granulométrie moyenne < 10 µm, à une température de 10 à 60°C et de préférence à la température ambiante, ce à l'occasion de quoi on subdivise en deux parties la quantité de ce mélange, rapportée aux matières de charge présentes, prévue pour incorporation dans le caoutchouc,
b) on mélange une première partie au latex polymère, à l'émulsion de polymères ou à la solution de polymères, on abaisse le pH de ce mélange avec un acide, en particulier un acide de Lewis, jusqu'à un pH de 6,0 à 4,5 (première partie, première étape),
c) on ajoute le reste (deuxième partie, partie dédoublée) , tout en continuant à abaisser le pH à une valeur de 4,5 à 2,6 et en particulier d'environ 3,2 (deuxième étape), de façon que le caoutchouc se trouvant dans le mélange se sépare par précipitation, en même temps que la matière de charge modifiée par le ou les composés organiques du silicium,
d) on sépare par des techniques connues en soi la matière solide qui a précipité,
e) de préférence, on la lave, pour ajuster le pH à une valeur d'environ 6 à 7, plus compatible avec le post-traitement, et
f) on sèche le caoutchouc contenant la matière de charge.

2. Procédé selon la revendication 1,
**caractérisé en ce que**
on utilise un ou plusieurs composés organiques du silicium de formule générale
[R¹ ₙ-(RO) ₃₋ₙ Si-(Alk)ₘ- (Ar)ₚ]_{q} [B] (I),
R¹ ₙ(RO)₃₋ₙ Si-(Alk) (II),
ou
R¹ ₙ(RO)₃₋ₙ Si-(alcényle) (III)
dans lesquelles
B : est -SCN, -SH, -Cl, -NH₂ (quand q = 1) ou -Sx- (quand q = 2)
R et R¹ : représentent chacun un groupe alkyle ayant de 1 à 4 atomes de carbone en chaîne droite ou ramifiée, le radical phényle, tous les radicaux R et R¹ pouvant chacun avoir des significations identiques ou des significations différentes, et de préférence un groupe alkyle,
R : est un groupe alkyle en C₁-C₄, alcoxy en C₁-C₄, à chaîne droite ou ramifiée,
n : vaut 0, 1 ou 2,
Alk : est un résidu carboné divalent à chaîne droite ou ramifiée ayant de 1 à 6 atomes de carbone,
m : vaut 0 ou 1,
Ar : est un résidu arylène ayant de 6 à 12 atomes de carbone,
p : vaut 0 ou 1, à la condition que p et m ne vaillent pas simultanément 0,
x : est un nombre de 2 à 8,
Alkyle : est un résidu hydrocarboné insaturé monovalent à chaîne droite ou ramifiée ayant de 1 à 20 et de préférence de 2 à 8 atomes de carbone,
Alcényle : est un résidu hydrocarboné insaturé monovalent à chaîne droite ou ramifiée ayant de 2 à 20 et de préférence de 2 à 8 atomes de carbone.

3. Procédé selon la revendication 1 ou 2,
**caractérisé en ce que**
on utilise en tant que substances tensioactives des tensioactifs non ioniques, cationiques ou anioniques.

4. Procédé selon la revendication 3,
**caractérisé en ce que**
on utilise les tensioactifs en une quantité de 1 à 15 % en poids, en particulier de 2 à 10 % en poids par rapport à la quantité des composés organosilanes dans l'émulsion.

5. Procédé selon l'une ou plusieurs des revendications précédentes,
**caractérisé en ce que**
la concentration du composé organique du silicium dans la suspension est de 0,3 à 15 % en poids par rapport à la matière de charge (sèche absolue).

6. Procédé selon la revendication 1,
**caractérisé en ce que**
on provoque la précipitation de la poudre de caoutchouc en présence d'un acide, de préférence un acide de Lewis, en particulier Al₂(SO₄)₃.

7. Procédé selon la revendication 3,
**caractérisé en ce que**
on utilise un tensioactif de la classe des alcools gras polyéthoxylés ou des alkylphénols polyéthoxylés.

8. Procédé selon l'une ou plusieurs des revendications précédentes,
**caractérisé en ce que**,
dans l'étape a), on utilise en tant que matière de charge en particulier le gâteau de filtration, lavé jusqu'à élimination des sels, d'une silice précipitée.

9. Procédé selon l'une ou plusieurs des revendications précédentes,
**caractérisé en ce que**
l'on utilise la matière solide obtenue lors du traitement de matière de charge naturelle, en particulier des argiles.

10. Procédé selon la revendication 1,
**caractérisé en ce que**,
dans la première partie selon l'étape b), on utilise une quantité ≥ 50 parties de la partie matière de charge prévue dans la poudre de caoutchouc.

11. Procédé selon la revendication 1,
**caractérisé en ce que**,
dans la première partie selon b), on utilise pour une quantité ≥ 80 parties de matière de charge phr (pour cent parties de caoutchouc) 90 à 99 % de la quantité de matière de charge prévue dans la poudre de caoutchouc.

12. Procédé selon l'une ou plusieurs des revendications précédentes,
**caractérisé en ce que**
on ajoute à l'émulsion/suspension selon l'étape a) ou au mélange selon l'étape b) un noir de carbone utilisable dans la technique du caoutchouc, en la quantité souhaitée.

13. Procédé selon la revendication 1,
**caractérisé en ce que**
on ajoute à l'émulsion, à la suspension ou à la solution selon le point a) de la revendication 1, ou au mélange produit selon les points b) et c), des auxiliaires de mise en oeuvre, des agents de protection contre le vieillissement, des activateurs et/ou des produits chimiques réticulants, et du soufre en les quantités usuelles, qui sont courants dans l'industrie du caoutchouc.

14. Caoutchoucs finement divisés (poudre de caoutchouc) préparés selon la revendication 1, qui contiennent en les quantités usuelles du caoutchouc, des matières de charge et des auxiliaires de mise en oeuvre, des agents de protection contre le vieillissement, des activateurs et/ou des produits chimiques réticulants et du soufre, courants dans l'industrie du caoutchouc.
